Europäisches Patentamt

European Patent Office

Office européen des brevets

(11) Veröffentlichungsnummer: **0 601 414 A1**

# (12) EUROPÄISCHE PATENTANMELDUNG

(21) Anmeldenummer: **93119088.8**

(22) Anmeldetag: **26.11.93**

(51) Int. Cl.5: **C07C 45/46**, C07C 45/47, C07C 45/48

(30) Priorität: **05.12.92 DE 4240965**

(43) Veröffentlichungstag der Anmeldung:
**15.06.94 Patentblatt 94/24**

(84) Benannte Vertragsstaaten:
**BE CH DE ES FR GB IT LI NL**

(71) Anmelder: **BASF Aktiengesellschaft
Carl-Bosch-Strasse 38
D-67063 Ludwigshafen(DE)**

(72) Erfinder: **Siegel, Wolfgang, Dr.
Spelzenstrasse 9
D-68167 Mannheim(DE)**
Erfinder: **Troetsch-Schaller, Irene, Dr.
Hannongstrasse 5
D-67227 Frankenthal(DE)**

(54) **Verfahren zur Herstellung von Acylaromaten.**

(57) Herstellung von Acylaromaten aus Aromaten und Carbonsäuren, dadurch gekennzeichnet, indem man die Ausgangsverbindungen mit Phosgen in Gegenwart eines aliphatischen Phosphinoxids oder eines N,N-Dialkylformamids sowie einer Fe(II)-, Fe(III)- oder Zn(II)-Verbindung umsetzt.

EP 0 601 414 A1

Die vorliegende Erfindung betrifft ein neues Verfahren zur Herstellung von Acylaromaten aus Aromaten und Carbonsäuren.

Für die Acylierung von Aromaten mit Carbonsäuren sind mehrere Kondensationsmittel bekannt.

Für solche Reaktionen werden mindestens zwei Äquivalente $AlCl_3$ bzw. 1,5 Äquivalente $ZnCl_2$ pro Äquivalent Carbonsäure benötigt (Houben-Weyl, Methoden der organischen Chemie, Thieme Verlag, Stuttgart 1973, Bd. 7/2a, S. 281 und 284). Diese überstöchiometrischen Mengen sind nicht nur unwirtschaftlich, sie führen bei der üblichen Aufarbeitung mit Wasser auch zu einer hohen Belastung der Produktionsabwässer durch Metall- und Chlorid-Ionen.

Reaktive Aromaten wie Phenole oder Phenolether können auch unter Verwendung einer Polyphosphorsäure im großen Überschuß acyliert werden (Houben-Weyl, loc. cit. S. 20 und 299). Ein Überschuß an Fluorwasserstoff als Kondensationsmittel wird in der GB 11 64 046 empfohlen.

Mindestens äquimolare Mengen an Lewissäuren wie Bor-, Titan-, Niob-, Tantal-, Phosphor-, Arsen- und Antimonhalogenide werden in der EP-A 69 598 in Kombination mit einer starken Säure als Kondensationsmittel beschrieben.

Eine bevorzugt stöchiometrische Menge einer Fluoralkansulfonsäure lehrt die EP-A 75 390 für die Herstellung von Benzoylaromaten.

Unterstöchiometrische Mengen an Sulfonsäuren genügen als Kondensationsmittel für die Herstellung von Arylketonen, wenn das während der Reaktion gebildete Wasser azeotrop aus dem Reaktionsgemisch entfernt wird (US-A 5 041 616). Bei dieser Reaktionsführung wird mit Wasser aufgearbeitet und das für die azeotrope Destillation benötigte Lösungsmittel muß entfernt werden.

Die DE-A 23 21 122 und DE-A 20 57 956 lehren die Umsetzung von Carbonsäuren mit Phosgen zu Carbonsäurehalogeniden in Gegenwart von aliphatischen Phosphinoxiden bzw. N,N-Dialkylformamiden.

Die Verwendung von katalytischen Mengen an Fe- und Zn-Verbindungen sowie von Jod für eine Acylierung von Aromaten mit Carbonsäurechloriden ist aus der Arbeit von Pearson et al. in Synthesis (1972) 531 bekannt.

Es bestand die Aufgabe, ein Verfahren bereitzustellen, daß die Acylierung von Aromaten mit Carbonsäuren erlaubt, ohne daß dazu stöchiometrische Mengen einer Lewissäure in Kombination mit einem Kondensationsmittel benötigt werden. Außerdem soll das Verfahren eine nicht hydrolytische Aufarbeitung der Reaktionsmischung ermöglichen.

Demgemäß wurde ein Verfahren zur Herstellung von Acylaromaten aus Aromaten und Carbonsäuren gefunden, das dadurch gekennzeichnet ist, daß man die Ausgangsverbindungen mit Phosgen in Gegenwart eines aliphatischen Phosphinoxids oder eines N,N-Dialkylformamids sowie einer Fe(II)-, Fe(III)- oder Zn(II)-Verbindung umsetzt.

Die folgende Reaktionsgleichung gibt die Reaktion schematisch wieder:

$$Ar-H + HO-CO-R + COCl_2 \rightarrow$$
$$Ar-CO-R + CO_2 + 2HCl$$

Ar = aromatischer Rest

R = organischer Rest

Die für die Reaktion in Betracht kommenden Aromaten umfassen Benzol, Naphthalin, Anthracen und andere polycyclische aromatische Kohlenwasserstoffe sowie ihre Derivate. Die bevorzugten Substituenten sind $C_1$-$C_{20}$-Alkylgruppen wie Methyl, Ethyl, Isopropyl, tert.-Butyl und tert.-Amyl, $C_6$-$C_{10}$-Arylgruppen wie Phenyl, $C_1$-$C_{20}$-Alkoxy wie Methoxy, Ethoxy, Isopropoxy, Aryloxy wie Phenoxy, $C_1$-$C_{20}$-Alkylthio wie Methylthio, Ethylthio und Propylthio, Hydroxy und Halogen wie Fluor, Chlor und Brom, Acylgruppen, die auch nach dem erfindungsgemäßen Verfahren eingeführt worden sein können, wie 2-Haloacyl, z.B. 2-Chloracetyl, 2-Chlorpropionyl, 2-Chlorbutanoyl oder Aroyl wie Benzoyl, 4-Chlorbenzoyl, 4-Fluorbenzoyl, 2-Methylbenzoyl, 3-Methylbenzoyl und 4-Methylbenzoyl. Die Aromaten können einen oder mehrere Substituenten tragen, die verschieden voneinander sein können.

Es seien hier Verbindungen genannt für welche das erfindungsgemäße Verfahren besondere Bedeutung hat:

Benzol, Toluol, o-Xylol, m-Xylol, p-Xylol, tert.-Butylbenzol, Diphenylether, Anisol, 1-tert.-Amyl-4-methylbenzol, 1,3-Dimethoxybenzol, 1-tert.-Butyl-4-methylbenzol, Chlorbenzol, 1,2-Dimethoxybenzol, 1,3,5-Trimethylbenzol, 1,2,4,5-Tetramethylbenzol.

Die im erfindungsgemäßen Verfahren eingesetzten Carbonsäuren unterliegen keinen erkennbaren Einschränkungen. Die bevorzugten Carbonsäuren sind jedoch aromatische Carbonsäuren, insbesondere Benzoesäure oder inerte Substituenten tragende Benzoesäuren. Diese inerten Substituenten umfassen $C_1$-

$C_{20}$-Alkyl wie Methyl, Ethyl, Propyl, $C_6$-$C_{10}$-Aryl wie Phenyl und Naphthyl, $C_1$-$C_{20}$-Alkoxy wie Methoxy, Ethoxy, iso-Propoxy, $C_6$-$C_{10}$-Aryloxy wie Phenoxy, Carboxy sowie Halogen wie Fluor, Chlor, Brom. Diese Substituenten können die 2-, 3- und/oder 4-Position der Carbonsäuren einnehmen. Es sind hier zu nennen: Benzoesäure, 2-, 3- und 4-Methylbenzoesäure, Phthalsäure, Isophthalsäure, Terephthalsäure, 2-, 3- und 4-Chlorbenzoesäure, 4-Methoxybenzoesäure, 4-Fluorbenzoesäure und 4-Phenoxybenzoesäure.

Weiterhin sind $\alpha$-Halogenalkansäuren bevorzugt wie Chloressigsäure, 2-Chlorpropionsäure, 2-Chlorbuttersäure, Bromessigsäure, 2-Brompropionsäure und 2-Brombuttersäure.

Die Reaktion findet in Gegenwart eines aliphatischen Phosphinoxids oder eines N,N-Dialkylformamids statt.

Die aliphatischen Phosphinoxide sind bevorzugt Trialkylphosphinoxide mit geradkettigen oder verzweigten $C_1$-$C_{12}$-Kohlenstoffresten. Besonders bevorzugt sind Tri-n-butylphosphinoxid, Tri-n-hexylund Tri-n-octylphosphinoxid.

Bei den N,N-Dialkylformamiden handelt es sich bevorzugt um solche mit $C_1$-$C_{30}$-Alkylketten, besonders bevorzugt um Di-sec.-butylformamid.

Weiterhin werden die definitionsgemäßen Metallverbindungen in der Reaktion eingesetzt. Es handelt sich um Fe(II)-Verbindungen wie $FeSO_4$, Fe(III)-Verbindungen wie $FeCl_3$, $FeBr_3$, $Fe_2(SO_4)_3$, Eisen(III)-Acetylacetonat, Eisen(III)-Carboxylate wie Benzoat und Acetat, und gemischtvalente Verbindungen wie $Fe_3O_4$ und bevorzugt um $Fe_2O_3$. Weiterhin kommen Zink(II)-Verbindungen wie $ZnCl_2$, $ZnBr_2$, $ZnO$ und Zinkcarboxylate wie Zinkbenzoat und Zinkacetat in Betracht.

Die Carbonsäure wird im allgemeinen in äquimolaren Mengen oder in geringem Überschuß oder Unterschuß in Bezug auf den Aromaten eingesetzt. Das bevorzugte molare Verhältnis beträgt 1 : 1 bis 0,8 : 1 für Carbonsäure zu Aromat.

Das Phosphinoxid bzw. das N,N-Dialkylformamid wird in der Regel in Mengen von 0,1 bis 5 mol-%, vorzugsweise 1 bis 2 mol-%, bezogen auf die Menge des Aromaten eingesetzt. Die Übergangsmetallverbindung wird im allgemeinen in Mengen von 0,1 bis 10 mol-%, vorzugsweise 1 bis 3 mol-% verwendet, bezogen auf die Menge des Aromaten.

Phosgen wird üblicherweise in stöchiometrischen Mengen oder in geringem molaren Überschuß bezogen auf die Menge der Carbonsäure, eingesetzt. Bevorzugt werden 1,05 : 1 bis 1,2 : 1 mol Phosgen pro Mol Carbonsäure.

In der praktischen Ausführung der Reaktion hat es sich bewährt, die Ausgangskomponenten Aromat und Carbonsäure mit dem aliphatischen Phosphinoxid oder dem N,N-Dialkylformamid sowie der Fe(II)-, Fe(III)- oder Zn(II)-Verbindung bei 100 bis 120°C vorzulegen und mit Phosgen zu versetzen. Überschüssiges Phosgen wird nach einer Reaktionszeit von etwa einer Stunde im Stickstoffstrom entfernt, und die Reaktion wird durch weiteres Erhitzen zu Ende geführt.

Die Reaktionstemperatur beträgt im allgemeinen 50 bis 200°C, bevorzugt 100 bis 180°C. Die Reaktion kann bei einem Überdruck bis etwa 10 bar ausgeführt werden, in der Regel wird sie aber bei Normaldruck vorgenommen. Die Reaktion ist im allgemeinen nach 4 bis 20 Stunden beendet. Sie kann in inerten Lösungsmitteln wie Nitrobenzol oder Kohlenwasserstoffen wie Heptan, Octan, Nonan, Decan oder deren Mischungen ausgeführt werden, bevorzugt wird sie aber ohne Lösungsmittel vorgenommen.

Die Isolierung der Reaktionsprodukte aus dem Ansatz ist durch bekannte Maßnahmen wie Kristallisation oder Destillation möglich.

Das erfindungsgemäße Verfahren erlaubt die Herstellung von Acylverbindungen aus Aromaten und Carbonsäuren in nur einer Stufe, wobei lediglich katalytische Mengen einer Lewissäure in Kombination mit Phosgen benötigt werden. Das Verfahren kann ohne Lösungsmittel ausgeübt werden. Die Isolierung der Verfahrensprodukte ist ohne eine Aufarbeitung mit Wasser möglich.

Die Verfahrensprodukte sind wichtige Zwischenprodukte, z.B. als Vorstufen für Farbstoffe, oder als Antioxidationsmittel.

Beispiele

Allgemeine Herstellvorschrift

1,2 mol eines $R^1$, $R^2$-disubstituierten Benzols, 1 mol einer substituierten Benzoesäure $R^3$-$C_6H_4$-COOH, 2 mol-% eines N,N-Dialkylformamids bzw. eines Phosphinoxids und 3 mol-% $Fe_2O_3$ wurden bei 100 bis 120°C vorgelegt. Es wurden 1,1 mol Phosgen eingeleitet. Nach einer Stunde wurde überschüssiges Phosgen im Stickstoffstrom entfernt, und die Reaktionslösung wurde für t Stunden auf eine Temperatur von T erhitzt. Die so erhaltenen Acylaromaten wurden durch Destillation oder Kristallisation aus Heptan gereinigt und isoliert.

Tabelle

| Bei-spiel | Benzol R$^1$ | R$^2$ | Benzoesäure R$^3$ | Zusatz | t [h] | T [°C] | Produkt (-benzophenon) | Aus-beute [%] |
|---|---|---|---|---|---|---|---|---|
| 1 | tert. Butyl | H | 2-Methyl | DsBF | 8 | 130 | 4-Butyl-2'-Methyl | 80 |
| 2 | tert. Butyl | H | 4-Methyl | TOPO | 10 | 140 | 4-Butyl-4'-methyl | 60 |
| 3 | Phenoxy | H | 2-Methyl | TOPO | 5 | 130 | 2-Methyl-4'-phenoxy | 75 |
| 4 | Phenoxy | H | 4-COOH | DsBF | 15 | 180 | 4-Carboxyl-4'-phenoxy | 50 |
| 5 | Cl | H | 2-Methyl | TOPO | 10 | 130 | 4-Chlor-2'-methyl | 15 |
| 6 | Methyl | H | H | TOPO | 15 | 160 | 4-Methyl | 75 |
| 7 | Methyl | H | 4-Methyl | TOPO | 15 | 160 | 4,4'-Dimethyl | 65 |
| 8 | Phenoxy | H | 4-Phenoxy | TOPO | 14 | 180 | 4,4'-Diphenoxy | 50 |
| 9 | Br | | 4-Methoxy | DsBF | 10 | 150 | 4-Brom-4'-methoxy | 20 |
| 10 | 1-Me | 4-Me | 4-Cl | DsBF | 15 | 145 | 2,5-Dimethyl-4'-chlor | 85 |
| 11 | Methoxy | H | 4-Methyl | DsBF | 9 | 180 | 4-Methoxy-4'-methyl | 88 |
| 12 | 1-Methoxy | 3-Methoxy | H | DsBF | 6 | 160 | 2,4-Dimethoxy | 55 |
| 13 | 1-tert.Amyl | 4-Me | H | DsBF | 6 | 160 | 5-tert.Amyl-2-Methyl | 55 |

Me = Methyl          DsBF = N,N-Di-sec.butylformamid

$^t$Bu = tert. Butyl          TOPO = Tri-n-octylphosphinoxid

**Patentansprüche**

1. Verfahren zur Herstellung von Acylaromaten aus Aromaten und Carbonsäuren, dadurch gekennzeichnet, daß man die Ausgangsverbindungen mit Phosgen in Gegenwart eines aliphatischen Phosphinoxids

4

oder eines N,N-Dialkylformamids sowie einer Fe(II)-, Fe(III)- oder Zn(II)-Verbindung umsetzt.

2. Verfahren nach Anspruch 1, dadurch gekennzeichnet, daß man die Reaktion in Gegenwart von Di-sec.-butylformamid vornimmt.

3. Verfahren nach Anspruch 1, dadurch gekennzeichnet, daß man die Reaktion in Gegenwart von Tri-n-butyl-, Tri-n-hexyl- oder Tri-n-octylphosphinoxid vornimmt.

4. Verfahren nach den Ansprüchen 1 bis 3, dadurch gekennzeichnet, daß man die Reaktion in Gegenwart von $Fe_2O_3$ vornimmt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| A | EP-A-0 178 184 (RAYCHEM CORPORATION) <br> * Seite 12 - Seite 18; Ansprüche * <br> --- | 1-3 | C07C45/46 <br> C07C45/47 <br> C07C45/48 |
| A | US-A-3 651 099 (H.L. PERLINGER ET AL.) <br> * das ganze Dokument * <br> --- | 1,2 | |
| A | US-A-2 773 903 (W.B.HARDY ET AL.) <br> * das ganze Dokument * <br> --- | 1,2 | |
| A | GB-A-717 720 (UNION CARBIDE AND CARBON CORPORATION.) <br> * das ganze Dokument * <br> ----- | 1 | |

|  | | | RECHERCHIERTE SACHGEBIETE (Int.Cl.5) |
|---|---|---|---|
| | | | C07C |

Der vorliegende Recherchenbericht wurde für alle Patentansprüche erstellt

| Recherchenort | Abschlußdatum der Recherche | Prüfer |
|---|---|---|
| DEN HAAG | 11. Januar 1994 | Bonnevalle, E |